# EUROPEAN PATENT SPECIFICATION

(11) **EP 4 420 602 B1**
(45) Date of publication and mention of the grant of the patent: **24.09.2025**
(21) Application number: 24159754.1
(22) Date of filing: 26.02.2024
(51) Int. Cl.: A61B 5/00, A61B 1/24, A61B 1/00

(54) **DENTAL PLAQUE DETECTION DEVICE**
VORRICHTUNG ZUR ERKENNUNG VON ZAHNBELAG
DISPOSITIF DE DÉTECTION DE PLAQUE DENTAIRE

(30) Priority: 27.02.2023 IT 202300003390
(43) Date of publication of application: 28.08.2024
(73) Proprietor: I.D.I. Evolution S.r.l., 20900 Monza (IT)
(72) Inventor: PIANTONI, Andrea, 20900 Monza (IT)
(74) Representative: Dragotti & Associati S.R.L.

(56) References cited:
- CN-U- 210 811 694
- CN-U- 218 247 473
- KR-A- 20190 105 333
- US-A1- 2013 189 641
- US-A1- 2019 328 234
- US-A1- 2021 153 747

## Description

The present invention relates to a dental plaque detection device.

It is well known that dental plaque is a sticky, transparent bacterial biofilm that settles on the surface of teeth. Although not visible to the naked eye, as it accumulates, it first compromises the natural white colour of the enamel by turning it yellow.

Secondly, it should be noted that saliva together with food residues and the composition of liquids contribute to the adhesion of plaque to the surface of the teeth. This deposit or plaque creeps into the interstitial spaces between the teeth and along the gumline. If neglected, plaque crystallises into tartar in which the mineralised bacterial load takes on a hard consistency and a yellowish or dark brown colour.

Tartar must be removed with the intervention of a dental hygienist using special devices.

The most common symptoms of dental plaque should not be underestimated, especially as they run the risk of worsening the health conditions of the oral cavity.

In fact, the presence of plaque and tartar encourages receding gums, the formation of gingival pockets and exposure of dentin to the point of tooth loss. This means that the accumulation of bacterial load promotes decay that can penetrate deep into the tooth pulp.

It is therefore necessary to know in good time whether tartar is present in order to be able to proceed with its removal, and this knowledge usually occurs for the first time at a dental appointment.

Scheduled appointments are used to improve this situation, but these also depend on the patient and his or her oral cavity care.

In these appointments, the dentist or dental hygienist introduces a small mirror into the patient's mouth in order to obtain a detailed view of the inner wall of the teeth and decide which and what type of intervention to perform.

This entails going to the dental surgery and performing this type of examination with wasted time for waiting and moving, and wasted time for the dentist as well.

It would therefore be desirable to have something available that allows the patient to detect his or her situation directly and that enables him or her to contact the dental hygienist to proceed with the removal of any deposits.

US 2021/153747 A1 discloses a mobile device for detecting an oral pathology with a probing unit that includes a fibre bundle set and a contact part configured to contact a gum portion of a tooth of an examinate.

US 2013/189641 A1 discloses a detection device e.g. for the detection of plaque, comprising a proximal end and a light-transmitting portion, wherein the proximal end comprises a light detection means and the light-transmitting portion comprises means for transmitting light along a path from a surface of interest to the light detection means.

The general object of the present invention is to provide a dental plaque detection device capable of solving the above-mentioned drawbacks of the prior art in an extremely simple, inexpensive and particularly functional manner.

Another object of the present invention is to provide a plaque detection device that can be used directly by the patient without the need to contact a specialist.

Another purpose of the present invention is to provide a dental plaque detection device that is able to display the deposited tartar in an easy-to-assess representation.

The above-mentioned purposes are achieved by a dental plaque detection device made according to the independent claim 1 and the following sub-claims.

The structural and functional characteristics of the present invention and its advantages with respect to the prior art will become even clearer and more evident from an examination of the following description, referring to the attached schematic drawings, which show an embodiment of the invention itself. In the drawings:

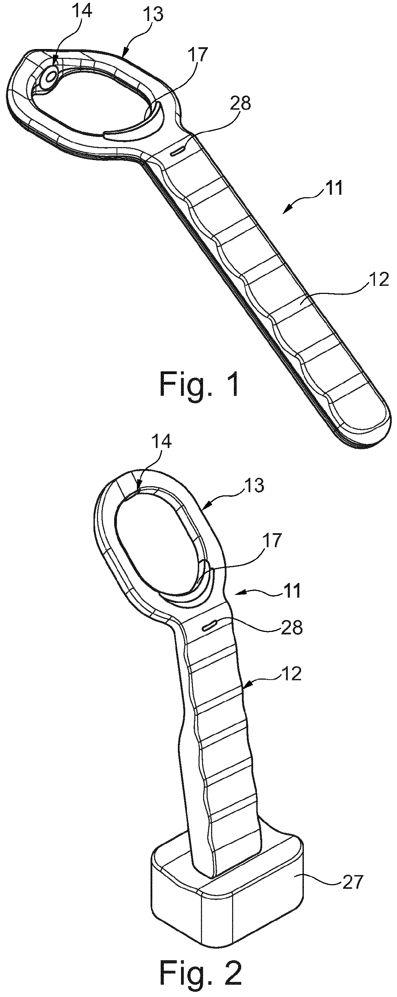

- Figure 1 is a perspective view illustrating a dental plaque detection device according to the present invention;
- Figure 2 is a further perspective view from a different angle of the dental plaque detection device in Figure 1 according to the invention when placed on a support base thereof;
- Figures 3, 4 and 5 are views of parts of the device in perspective and side elevation;
- Figure 6 is a perspective view illustrating a device according to the invention arranged in a representation of a mouth and teeth with teeth closed onto it in the detection position;

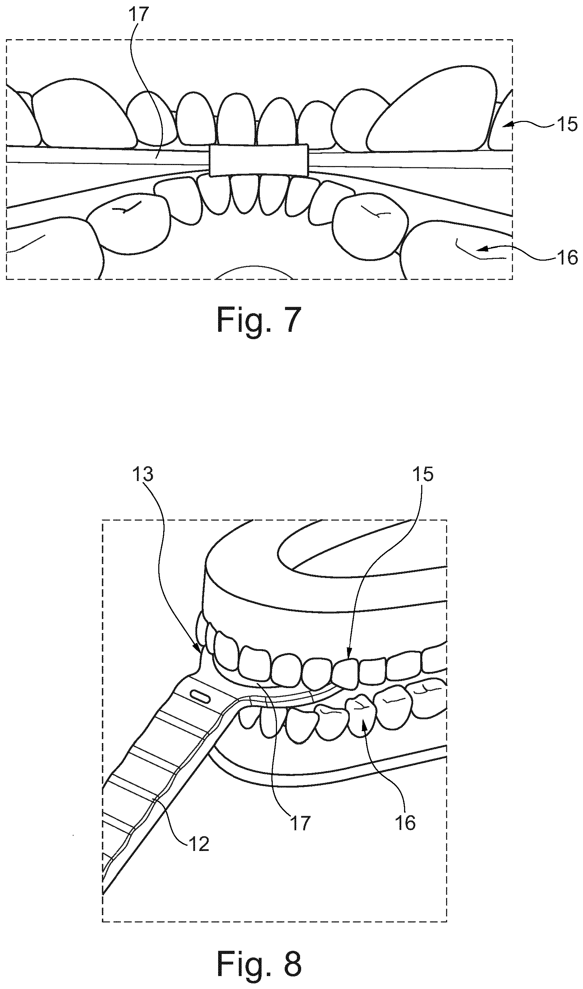

- Figure 7 is an enlarged view of a support block that is part of the device where the top and bottom teeth are resting in the detection position of Figure 6;
- Figure 8 is a perspective view similar to Figure 6 with a device according to the invention arranged in a representation of a mouth and teeth with teeth closed onto it in the detection position with a portrayal of a hand activating the detection button;
- Figure 9 is a perspective view illustrating a mobile phone screen on which the scan carried out by the dental plaque detection device according to the present invention has been received;
- Figure 10 shows parts of the shaped holder and handle of the device according to the invention where parts relevant to its operation are arranged.

With reference to the figures, a dental plaque detection device made according to the present invention is shown in an illustrative and non-limiting embodiment.

A dental plaque detection device according to the invention is collectively referred to as 11 and comprises a handle 12 extending into a shaped holder 13 bearing an image detector system 14.

The device as an assembly comprising the handle 12 and shaped holder 13 identifies a waterproof and durable casing, e.g. made of injection-moulded plastic.

This image detector system 14 is placed almost at one end of the shaped holder 13, opposite and facing the connection of the holder 13 to the handle 12 of the device.

Opposite the image detector system 14, in a final intermediate portion of the handle 12, there is a support block 17. The support block 17 defines a position in the dental plaque detection device 11 at a predetermined distance D from the image detector system 14.

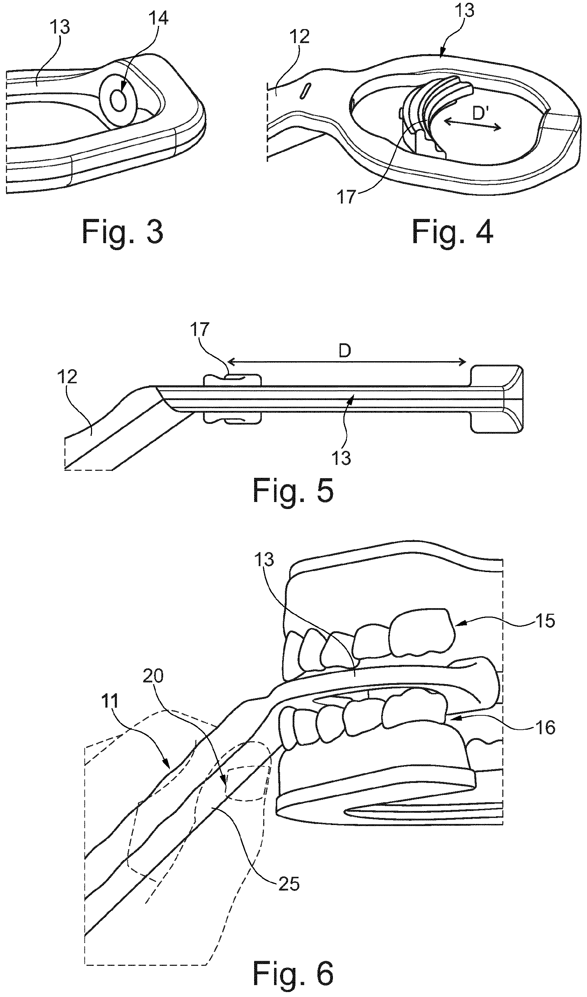

Figures 4 and 5 show how the support block 17 can be moved within the shaped holder 13 from a first position, at a first predetermined distance D from the image detector system 14, to a second position, at a second D' or more predetermined distances from the image detector system 14.

This feature is particularly advantageous in use in order to be able to vary the position of the support block 17, i.e., the point of support of the teeth, depending on the size of the mouth and the arrangement of the teeth within it, and to allow the image detection system 14 to produce coherent images.

According to the invention, this shaped holder 13 is made in the form of an annular element.

In the present description, the term "annular element" refers to an element comprising a perimeter wall defining an empty space within it. In this sense, the perimeter extension of such an element can be either curvilinear or polygonal as long as its perimeter wall surrounds an empty space. For example, the plan view of such an element may be circular, oval, elliptical, but also triangular, square, pentagonal, etc.

The main advantage of an annular element, as defined above, is to facilitate the "work" of the optical unit as it leaves the view of the teeth inside the mouth completely free as opposite and facing the optical unit arranged at the opposite end of the shaped holder 13.

Since the shaped holder 13 is configured to be inserted into a patient's mouth, a curved perimeter extension, e.g. oval-shaped, as illustrated in the accompanying figures, is preferable as it favours its insertion by having an almost spoon-like shape.

The image detector system 14 comprises an optical unit 18 associated with a processor 19 adapted to receive and possibly process images taken by the optical unit 18. Further, the processor 19 is enabled for wireless communications, e.g. by incorporating a Wi-Fi-enabled network module or any variant of the IEEE 802.11 protocol, and is adapted to transmit images to an external receiver integrated into a PC or phone, or other "smart device", in a dedicated app or other receiving element.

The image detector system 14 can be activated by the patient through an actuator 20 placed on the detection device 11.

The actuator 20, in a first embodiment, is made in the form of a button located at the bottom of t handle 12 (Figure 6) and operable by a single press of a finger, or, in a second embodiment, it is incorporated into the support block 17 and operable with the pressure of the patient's teeth on it.

Naturally, different ways of implementing the image detector system 14 are also possible, such as, but not limited to, a proximity sensor placed on the detection device 11.

The optical unit 18 of the image detector system 14 may be a camera applied to the shaped holder 13, as stated opposite and facing the handle 12 of the device or support block 17.

Preferably the camera 18 is equipped with a wide-angle lens necessary for the static configuration with a field of view >120°.

Coupled with the optical unit 18 in the form of a camera or/and incorporated into it are LED lights (UVA and/or white light), depicted as 22, or even additional lighting to detect and capture any "problems" with the teeth. In particular, UVA is useful for detecting tartar and mature plaque, while white lighting is useful for general tooth problems.

As mentioned above, the processor 19 associated with the optical unit 18 receives, and if necessary processes, the images and has the option of sending them to an external receiver such as a PC or telephone in an app or other receiving element. This envisages wireless local area network (WLAN) technologies that allow several devices (e.g. personal computers, smartphones, smart TVs, phones, etc.) to be connected to each other via radio waves and exchange data, in this case the images detected by the optical unit 18.

The flow of images in the form of data can be directed to a phone (Wi-Fi-direct) or through a local router, known in itself.

The plaque detection device 11 also comprises a battery 21 to which the image detector system 14, and thus the camera or optical unit 18, the processor 19 and the lighting source 22 are connected.

The battery 21 may be of the rechargeable type, and for this purpose the device will have a charging port 22 associated with it. Typically, a LiPo-type battery with a minimum capacity of 750 mAh may be required to operate such an arrangement.

The device may include a battery management system 21, of the activation button 20 of the image detector system 14 as well as a reset button 25. The management system also includes a charging port 26 located on the handle 12 and connectable to a separate support base 27 on which the device 11 is placed. The support base 27 is separate from the device and the device is placed on it at rest and/or in charging mode.

The detection device according to the invention, for example, can be positioned in the bathroom on the charging support base 27 and has an indicator light 28 that lights up when it is time to scan again. That is, when the indicator light turns green.

When this happens, it is possible to use the detection device, which is grasped with one hand and placed inside the mouth, positioned correctly as shown in Figure 6. This position must be such that on the support block 17 provided in the device, both the upper 15 and the lower 16 part of the teeth are arranged so that the correct distance of the teeth from the image detector system 14 can be identified in order to make correct and coherent images.

If the position is not correct, it is possible for the support block 17 to be moved inside the shaped holder 13 so that the support point of the teeth can be varied according to the size of the mouth and the arrangement of the teeth inside it. (Figures 4 and 5)

Once this position has been reached, the patient must take action and press the button 20 on the device to allow the image detector system 14 to acquire an image, i.e. scan the inside of the teeth.

During this step, the support block 17 keeps the bottom teeth 15 and the top teeth 16 at a predetermined distance from the image detector system 14, allowing the image detector system 14 to acquire a sharp, non-blurred image, free from defects due to any unintentional movement of the teeth by the user.

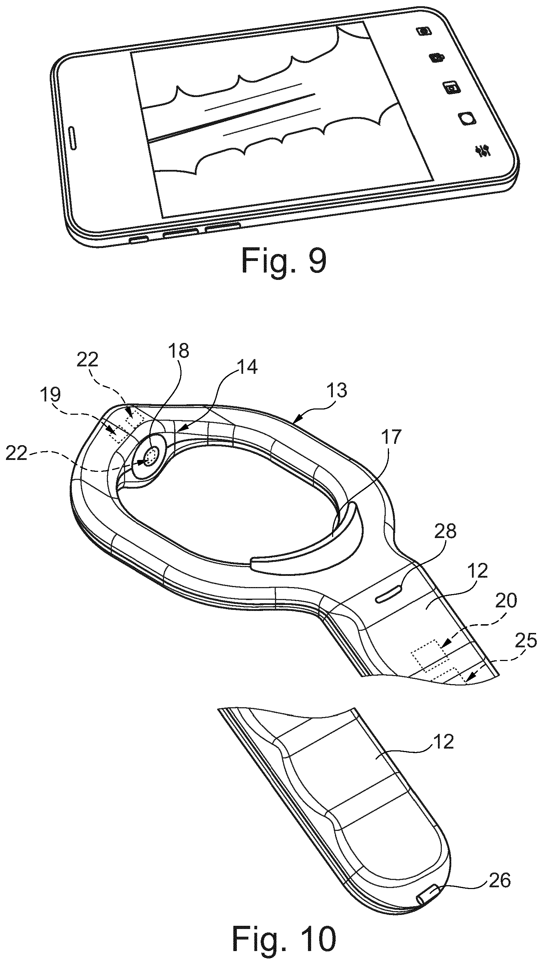

Since the optical unit 18 of the detector is associated with the processor 19, the latter receives and processes the images and has the possibility to send them to an external receiver such as a PC or phone (Figure 9 shows a perspective view illustrating a mobile phone screen on which the scan carried out by the dental plaque detection device according to the present invention has been received) by means of an app or other receiving element.

In this way, the patient immediately has a picture of his or her dental and tartar situation and can immediately contact the dentist if necessary.

The mobile phone, or equivalent device, can in fact display a detailed image of the scan performed by the image detector system 14, with an overview of the plaque level, and/or the position of plaque on each tooth. According to a particularly advantageous characteristic, an application on the phone is also able to provide the user with additional information on the basis of the acquired image, such as, for example, personalised dental care suggestions.

By gaining an overview of plaque accumulation over time, it is even possible to make a prediction of when the next dental procedure will be needed.

The dental plaque detection device made according to the present invention therefore allows for immediate and simple knowledge of plaque by the persons directly concerned.

The image is also useful and immediate to send and submit to the dentist, who visualises where plaque is causing problems and schedules the appropriate and necessary appointments in the surgery.

With the use of a dental plaque detection device made according to the present invention, an undoubted improvement in dental health is achieved, overcoming patients' reluctance and laziness.

Naturally, the shapes of the structure of a dental plaque detection device made according to the present invention may differ from those shown as non-limiting examples in the drawings, as may the materials and assembly methods.

The object mentioned in the preamble of the description is thus achieved.

The scope of protection of the present invention is defined by the accompanying claims.

## Claims

1. A dental plaque detection device comprising a handle (12), which extends into a shaped holder (13), and an image detector system (14) arranged in the shaped holder (13), wherein
- said image detector system (14) is placed at a free end of the shaped holder (13), opposite and facing the connection of the shaped holder (13) to the handle (12),
- said image detector system (14) comprises an optical unit (18) and a processor (19) associated with said optical unit (18);
- said image detector system (14) being connected to an actuator (20) placed on the detection device (11) and configured to be activated by a patient through said actuator (20); whereby
- the detection device (11) further comprises a support block (17), adapted to act as a support for an upper part (15) and a lower part (16) of a patient's teeth, wherein said support block (17) is placed in said shaped holder (13) opposite and facing said image detector system (14), and
- said shaped holder (13) is made in the form of an annular element attached to said handle (12).

2. Device according to claim 1, **characterised in that** said support block (17) is movable in position within said shaped holder (13).

3. Device according to claim 1, **characterised in that** said shaped holder (13) in the form of an annular element is oval shaped.

4. Device according to one or more of the preceding claims, **characterised in that** said optical unit (18) is a camera applied in the shaped holder (13).

5. Device according to claim 4, **characterised in that**, said camera (18) it is equipped with a wide-angle lens necessary for static configuration with a field of view >120°.

6. Device according to one or more of the preceding claims, **characterised in that** said image detector system (14) is coupled with UVA and/or white LED lights (22).

7. Device according to one or more of the preceding claims, **characterised in that** said handle (12) is provided with a charging port (26) connectable to a support base (27) separate from the device and on which the device is placed at rest and/or in charging mode.

8. Device according to one or more of the preceding claims, **characterised in that** it further comprises a battery (21).

9. Device according to one or more of the preceding claims, **characterised in that** the processor (19) is enabled for wireless communications.

## Patentansprüche

1. Vorrichtung zur Erkennung von Zahnbelag, umfassend einen Handgriff (12), der sich in eine geformte Halterung (13) erstreckt, und ein Bilddetektorsystem (14), das in der geformten Halterung (13) angeordnet ist, wobei
- das Bilddetektorsystem (14) an einem freien Ende der geformten Halterung (13) gegenüber und zugewandt der Verbindung der geformten Halterung (13) mit dem Handgriff (12) angeordnet ist,
- das Bilddetektorsystem (14) eine optische Einheit (18) und einen der optischen Einheit (18) zugeordneten Prozessor (19) umfasst;
- das Bilddetektorsystem (14) mit einem auf den Detektionsgerät (11) angeordneten Aktuator (20) verbunden ist und so konfiguriert ist, dass es durch einen Patienten über den Aktuator (20) aktiviert werden kann;
wobei
- das Detektionsgerät (11) ferner einen Stützblock (17) umfasst, der als Stütze für einen oberen Teil (15) und einen unteren Teil (16) der Zähne eines Patienten dient, wobei der Stützblock (17) in der geformten Halterung (13) gegenüber und gegenüber dem Bilddetektionssystem (14) angeordnet ist, und
- die geformte Halterung (13) in Form eines ringförmigen Elements ausgebildet ist, das an dem Handgriff (12) befestigt ist.

2. Gerät nach Anspruch 1, **dadurch gekennzeichnet, dass** der Stützblock (17) innerhalb der geformten Halterung (13) in seiner Position bewegbar ist.

3. Gerät nach Anspruch 1, **dadurch gekennzeichnet, dass** die geformte Halterung (13) in Form eines ringförmigen Elements oval geformt ist.

4. Gerät nach einem oder mehreren der vorstehenden Ansprüche, **dadurch gekennzeichnet, dass** die optische Einheit (18) eine in der geformten Halterung (13) angebrachte Kamera ist.

5. Gerät nach Anspruch 4, **dadurch gekennzeichnet, dass** die Kamera (18) mit einem für die statische Konfiguration erforderlichen Weitwinkelobjektiv mit einem Sichtfeld >120° ausgestattet ist.

6. Gerät nach einem oder mehreren der vorstehenden Ansprüche, **dadurch gekennzeichnet, dass** das Bilddetektorsystem (14) mit UVA- und/oder weißen LED-Leuchten (22) gekoppelt ist.

7. Gerät nach einem oder mehreren der vorstehenden Ansprüche, **dadurch gekennzeichnet, dass** der Handgriff (12) mit einer Ladebuchse (26) versehen ist, die mit einer von dem Gerät getrennten Halterung (27) verbunden werden kann, auf der das Gerät in Ruhe- und/oder Lademodus abgestellt wird.

8. Gerät nach einem oder mehreren der vorstehenden Ansprüche, **dadurch gekennzeichnet, dass** sie außerdem eine Batterie (21) umfasst.

9. Gerät nach einem oder mehreren der vorstehenden Ansprüche, **dadurch gekennzeichnet, dass** der Prozessor (19) für drahtlose Kommunikation ausgelegt ist.

## Revendications

1. Dispositif de détection de la plaque dentaire comprenant une poignée (12), qui s'étend dans un support façonné (13), et un système (14) de détection d'images disposé dans le support façonné (13), dans lequel
- le système (14) de détection d'images étant placé à une extrémité libre du support façonné (13), à l'opposé et en face de la connexion du support façonné (13) à la poignée (12),
- le système (14) de détection d'images comprenant une unité optique (18) et un processeur (19) associé à ladite unité optique (18) ;
- le système (14) de détection d'images étant connecté à un actionneur (20) placé sur le dispositif de détection (11) et configuré pour être activé par un patient par l'intermédiaire dudit actionneur (20) ;
dans lequel
- le dispositif de détection (11) comprend en outre un bloc de support (17), adapté pour servir de support à une partie supérieure (15) et à une partie inférieure (16) des dents d'un patient, dans lequel ledit bloc de support (17) est placé dans ledit support de forme (13) à l'opposé et en face dudit système (14) de détection d'images, et
- le support façonné (13) se présentant sous la forme d'un élément annulaire fixé à ladite poignée (12).

2. Dispositif selon la revendication 1, **caractérisé en ce que** ledit bloc de support (17) est mobile en position à l'intérieur dudit support façonné (13).

3. Dispositif selon la revendication 1, **caractérisé en ce que** ledit support façonné (13) sous forme d'élément annulaire est de forme ovale.

4. Dispositif selon l'une ou plusieurs des revendications précédentes, **caractérisé en ce que** ladite unité optique (18) est une caméra appliquée dans le support façonné (13).

5. Dispositif selon la revendication 4, **caractérisé en ce que** ladite caméra (18) est équipée d'un objectif grand angle nécessaire pour une configuration statique avec un champ de vision >120°.

6. Dispositif selon l'une ou plusieurs des revendications précédentes, **caractérisé en ce que** ledit système (14) de détection d'images est couplé à des lampes LED UVA et/ou blanches (22).

7. Dispositif selon l'une ou plusieurs des revendications précédentes, **caractérisé en ce que** ladite poignée (12) est pourvue d'un port de charge (26) connectable à une base de support (27) distincte de l'appareil et sur laquelle l'appareil est placé au repos et/ou en mode de charge.

8. Dispositif selon l'une ou plusieurs des revendications précédentes, **caractérisé en ce qu'**il comprend en outre une batterie (21).

9. Dispositif selon l'une ou plusieurs des revendications précédentes, **caractérisé en ce que** le processeur (19) est apte aux communications sans fil.
